# FASCICULE DE BREVET EUROPEEN

(11) **EP 2 588 074 B1**
(45) Date de publication et mention de la délivrance du brevet: **01.03.2017**
(21) Numéro de dépôt: 11741612.3
(22) Date de dépôt: 28.06.2011
(51) Int. Cl.: A61K 8/64, A61K 38/08, A61Q 17/04, A61Q 19/00, A61Q 19/08, C07K 7/06, C07K 14/47

(54) **PEPTIDES ACTIVATEURS DE LA SIRTUINE 6 ET COMPOSITIONS COSMETIQUES OU PHARMACEUTIQUES LES COMPRENANT**
SIRTUIN 6 AKTIVIERENDE PEPTIDE UND DEREN VERWENDUNG ZUR HERSTELLUNG KOSMETISCHER ODER PHARMAZEUTISCHER ZUSAMMENSETZUNGEN
SIRTUIN 6 ACTIVATOR PEPTIDES AND COSMETIC OR PHARMACEUTICAL COMPOSITIONS CONTAINING THE SAME

(30) Priorité: 29.06.2010 FR 1002698
(43) Date de publication de la demande: 08.05.2013
(73) Titulaire: ISP Investments Inc., Wilmington, DE 19805 (US)
(72) Inventeur: DAL FARRA, Claude, Kerhonkson NY 12446 (US); DOMLOGE, Nouha, F-06560 Valbonne (FR); BOTTO, Jean-Marie, F-06560 Valbonne (FR); IMBERT, Isabelle, 06400 Cannes (FR); PERNODET, Nadine, Huntington Station, NY 11746 (FR)
(74) Mandataire: Macquet, Christophe
(86) Numéro de dépôt international: PCT/FR2011/000373
(87) Numéro de publication internationale: WO 2012/001245

(56) Documents cités:
- WO-A2-2006/103354
- BERGERON L. ET AL.: "The effect of TRF2 telomeric protein modulation in an in vitro model of replicative and accelerated senescence", J. INVEST. DERMATOL., vol. 130, no. Suppl.1, avril 2010 (2010-04), page S76, XP009144757,
- MICHISHITA E. ET AL.: "SIRT6 is a histone H3 lysine 9 deacetylase that modulates telomeric chromatin", NATURE, vol. 452, no. 7186, 27 mars 2008 (2008-03-27), pages 492-496, XP002623499, cité dans la demande
- SCHLICKER C. ET AL.: "Substrates and Regulation Mechanisms for the Human Mitochondrial Sirtuins Sirt3 and Sirt5", J. MOL. BIOL., vol. 382, no. 3, 2008, pages 790-801, XP025610001,
- AMOYEL A. ET AL.: "Sirt6 play an essential anti aging role in skin cells", J. INVEST. DERMATOL., vol. 130, no. Suppl.2, 1 septembre 2010 (2010-09-01), page S29, XP009144633,

## Description

### Domaine de l'invention

La présente invention se situe dans le domaine cosmétique et pharmaceutique, et plus particulièrement dans le domaine de la dermatologie. La présente invention se rapporte à des peptides activateurs de la sirtuine 6 (SIRT6), dérivés de régions hautement conservées des protéines SIRT humaines.

La présente invention concerne également une composition cosmétique ou pharmaceutique, comprenant un peptide activateur de la SIRT6, utilisé seul ou en association avec au moins un autre agent actif, dans un milieu physiologiquement acceptable. L'invention est également relative à l'utilisation de ce nouveau peptide, en tant qu'agent actif, dans une composition cosmétique. L'invention concerne encore l'utilisation d'une composition cosmétique pour prévenir et/ou réparer les dégradations de l'ADN, améliorer la maintenance des télomères et diminuer la sénescence cellulaire. L'invention porte enfin sur un procédé de traitement cosmétique destiné à prévenir et/ou traiter les signes cutanés du vieillissement et du photo-vieillissement, selon lequel on applique une quantité efficace d'agent actif, ou une composition le contenant, sur les zones à traiter.

### Arrière-plan de l'invention

Le vieillissement correspond à l'ensemble des processus physiologiques qui modifient la structure et les fonctions de l'organisme en fonction du temps et des agressions subies. On distingue le vieillissement intrinsèque dû à des facteurs génétiques, à des modifications biochimiques qui ont lieu lors d'états de fatigue, de stress, de changements hormonaux comme lors de la grossesse, etc., et le vieillissement extrinsèque dû à des facteurs environnementaux auxquels est soumis l'organisme tout au long de sa vie, tels que la pollution, le soleil, les maladies, les habitudes de vie, etc. Le vieillissement est un processus lent et progressif qui touche toutes les cellules et tous les organes. Il en va ainsi de la peau qui constitue une barrière entre l'environnement extérieur et le milieu intérieur et protège l'organisme contre les agressions extérieures. Au cours du vieillissement l'aspect de la peau est modifié et l'on voit apparaître alors des rides et ridules, des tâches d'hyper ou d'hypo-pigmentation, une sècheresse voire une déshydratation de la peau, un amincissement de l'épiderme, une élastose, etc.

Le vieillissement intrinsèque est étroitement lié aux divisions répétées des cellules. Ainsi, dans les cellules somatiques humaines, les télomères raccourcissent au rythme des divisions cellulaires, jusqu'à l'apparition de télomères dysfonctionnels qui induisent la sénescence ou l'apoptose selon le type cellulaire. Ce phénomène constitue l'horloge biologique qui explique le fait que les cellules somatiques humaines sont programmées pour un nombre limité de division.

Les phénomènes de sénescence cellulaire sont accélérés par les dommages oxydatifs, en particulier dans les zones du corps où la peau est exposée au soleil ; le photo-vieillissement se superpose alors au vieillissement intrinsèque. Les dommages oxydatifs sont favorisés par divers agents aussi bien endogènes (métabolisme, inflammation, cycles redox) qu'exogènes, comme les rayonnements UV et les radiations ionisantes, le tabagisme et diverses molécules apportées par l'alimentation (métaux toxiques, alcool). Les dommages provoqués par le stress oxydatif atteignent aussi bien l'ADN que les lipides et les protéines. Au niveau de l'ADN, le stress oxydatif provoque de nombreuses modifications structurales (mutations, coupures, pontages covalents de protéines). Les bases oxydées, comme la 8-oxo-guanine, augmentent avec l'âge et peuvent atteindre jusqu'à 10 000 bases par jour et par cellule.

Pour lutter contre le vieillissement, il est donc intéressant d'identifier de nouveaux composés capables à la fois de lutter contre les dommages ponctuels causés à l'ADN par les stress oxydatifs et de ralentir la sénescence cellulaire en favorisant la stabilité des télomères.

Or les inventeurs ont récemment identifié une cible moléculaire intéressante, capable de remplir ces différentes fonctions.

Les protéines SIRT sont des protéines nucléaires ou mitochondriales, portant une fonction désacétylase NAD+ dépendantes et appartenant à la famille des Sirtuines. L'activité déacétylase ou mono-ADP-ribosyltransférase des sirtuines leur permet de moduler le niveau d'acétylation de certaines histones, ce qui suggère leur implication, notamment pour les sirtuines 1, 2 et 3, dans la régulation de phénomènes épigénétiques.

La famille des sirtuines humaines comprend 7 protéines, très conservées au cours de l'évolution, dénommées SIRT1 à SIRT7.

La SIRT6 est une sirtuine nucléaire associée spécifiquement à la chromatine des télomères et qui joue un rôle dans la maintenance et à la stabilisation des structures télomériques (Michishita et al. Nature. 2008 Mar 27 ; 452(7186):492-6). Ainsi, chez la souris invalidée pour le gène SIRT6, on observe un vieillissement prématuré et une durée de vie raccourcie, ainsi qu'une augmentation de la sénescence réplicative des kératinocytes (Kawahara TL et al. Cell. 2009 Jan 9;136(1):62-74).

Les télomères sont des structures qui coiffent l'extrémité des chromosomes et les protègent contre la dégradation enzymatique, la recombinaison et les fusions interchromosomiques. Chez l'homme ces structures sont constituées d'une séquence d'ADN répétée des milliers de fois, associée à des protéines spécifiques, telles que TRF1 et TRF2. Des études récentes ont montré que l'expression de TRF2 décline lors du vieillissement des cellules (Amoyel et al., J. Invest. Dermatol. Apr 2009; 129 (Supplement 1 s), s70).

D'autre part SIRT6 joue un rôle important dans la réparation de l'ADN par excision de bases, un mécanisme de réparation de l'ADN utilisé par la cellule lorsque l'ADN a été endommagé par des oxydants. Ces découvertes suggèrent que SIRT6 est nécessaire à la régulation de l'intégrité génomique et des phénomènes de vieillissement et pourrait être directement impliqué dans l'augmentation de la longévité cellulaire (Mostoslavsky et al., Cell. 2006 Jan 27;124(2):315-29).

Il est connu que l'utilisation de peptides activateurs de la protéine SIRT1 (FR 2883751, FR 2883752, FR 2883753, FR 2883754), permet de préparer des compositions cosmétiques ou pharmaceutiques utile pour protéger la peau et lutter contre le vieillissement, ou encore que certains composés pharmaceutiques inducteurs de SIRT7 sont utiles pour traiter les maladies liées à l'âge (EP 1955715). Toutefois, à ce jour, aucun composé peptidique capable d'activer la protéine SIRT6 dans les cellules de la peau n'a été décrit, alors que le besoin de soin de la peau de ce type existe.

### Exposé de l'invention

Les inventeurs ont mis en évidence que des peptides dérivés de régions hautement conservées des protéines SIRT humaines de formule générale (I) suivante :

R₁-(AA)ₙ-X₁-X₂₋-X₃-X₄-X₅-X₆-(AA)ₚ-R₂

étaient de très bons agents activateurs de la SIRT6, et permettaient de prévenir et/ou réparer efficacement les dégradations de l'ADN provoquées par des agressions extérieures et en particulier par les rayonnements UV, d'améliorer la maintenance des télomères et de diminuer la sénescence cellulaire. En conséquence, ces peptides sont adaptés pour lutter contre le vieillissement et le photo-vieillissement de la peau.
Les peptides selon l'invention se caractérisent par le fait qu'ils :
- activent l'expression de la SIRT6 dans les cellules cutanées,
- diminuent les dégradations de l'ADN des cellules de la peau soumises à des rayonnements UVB,
- favorisent la protection des cellules de la peau soumises à des stress oxydatifs
- stimulent l'expression de la protéine TRF2, spécifiquement associée aux télomères,
- augmentent l'expression des protéines de la matrice extracellulaire par les fibroblastes,
- optimisent la fonction barrière de l'épiderme.

On entend par « peptide ou agent actif activateur de la SIRT6 ou capable d'activer la SIRT6 humaine», tout peptide de formule générale (I) capable d'augmenter la quantité de SIRT6 présente dans la cellule, soit en augmentant la synthèse protéique par modulation directe ou indirecte de l'expression génique, soit par d'autres processus biologiques tels que la stabilisation de la protéine ou encore la stabilisation des transcrits d'ARN messager.

On entend par peau, l'ensemble des tissus de recouvrement constituant la peau, les muqueuses et les phanères.

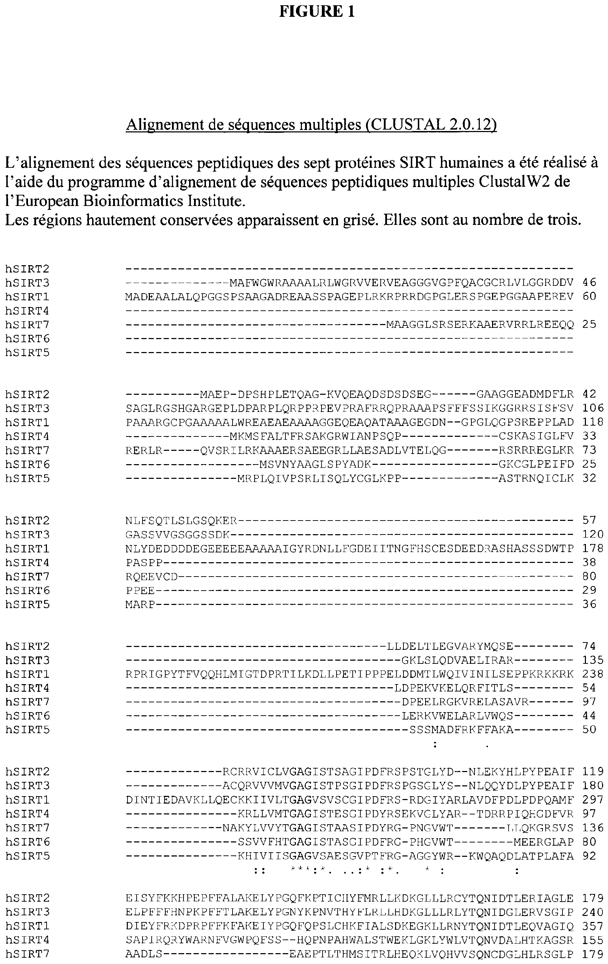

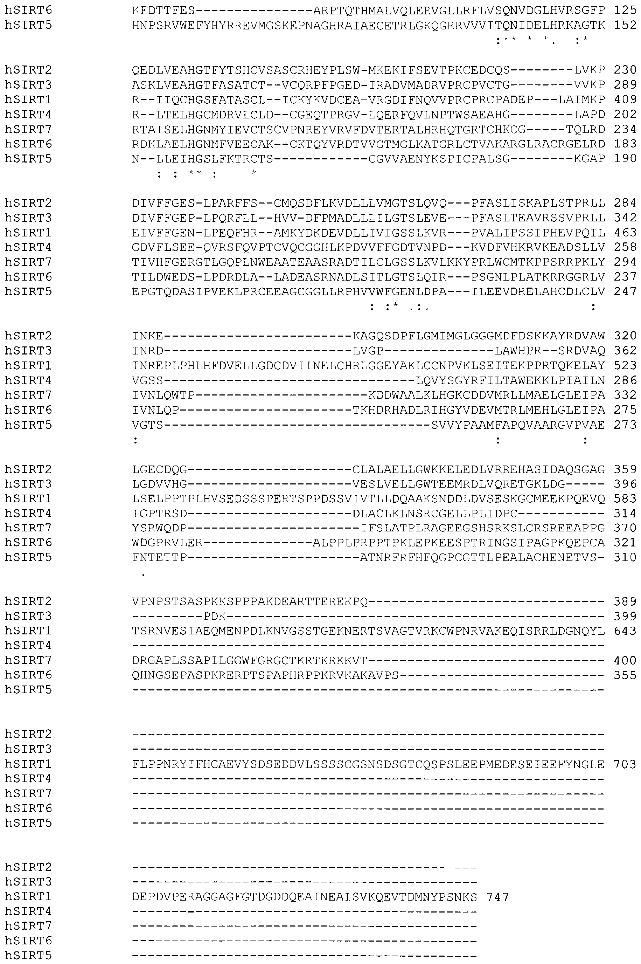

L'alignement des séquences peptidiques des 7 protéines de la famille des SIRT a été réalisé à l'aide du programme d'alignement de séquences peptidiques multiples ClustalW2 de l'European Bioinformatics Institute présentée dans la figure 1. L'alignement optimum fait apparaitre trois régions hautement conservées.

On entend par « région hautement conservée des protéines SIRT humaines », des séquences peptidiques comportant au moins 2 acides aminés consécutifs absolument identiques dans les 7 sirtuines de la famille, lorsque les séquences ont été alignées sur la base de la plus grande homologie.

La 1ere région hautement conservée comporte la séquence peptidique Gly-Ala-Gly,
La 2eme région hautement conservée comporte la séquence peptidique Gln-Asn,
La 3eme région hautement conservée comporte la séquence peptidique His-Gly.

Ainsi, l'invention a pour objet premier un peptide de 7 acides aminés, dérivé de la séquence peptidique d'une région hautement conservée des protéines SIRT humaines et qui est de
(SEQ ID n°4) Gly-Ala-Gly-Val-Ser-Ala-Glu
(SEQ ID n°5) Gly-Ala-Gly-Val-Ser-Ala-Glu-NH₂
(SEQ ID n°6) Thr-Gln-Asn-Ile-Asp-Glu-Leu
(SEQ ID n°7) Thr-Gln-Asn-Ile-Asp-Glu-Leu-NH₂

Selon un mode de réalisation particulièrement intéressant, le peptide correspond à la séquence SEQ ID n°4 ou à la séquence SEQ ID n°5.

Selon un autre mode de réalisation particulièrement intéressant, le peptide correspond à la séquence SEQ ID n°6 ou à la SEQ ID n°7.

Les acides aminés, peuvent être sous configuration isomérique L- et D-. De manière préférentielle, les acides aminés sont sous forme L.

Le terme « peptide » désigne un enchaînement de deux ou plusieurs acides aminés liés entre eux par des liaisons peptidiques ou par des liaisons peptidiques modifiées.

Par « peptide », il faut également entendre le peptide naturel ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

Les dérivés de peptides concernent notamment les acides aminés reliés entre eux par une liaison pseudo-peptidique. On entend par « liaison pseudo-peptidique », tous les types de liaisons susceptibles de remplacer les liaisons peptidiques « classiques ».

De façon à améliorer la résistance à la dégradation, il peut être nécessaire d'utiliser une forme protégée du peptide selon l'invention. De préférence, on utilise pour protéger la fonction amine primaire de l'acide aminé N-terminal, une substitution par un groupement R₁ de type acyle possédant une chaîne alkyle de C₁ à C₃₀ saturée ou insaturée, pouvant être choisie parmi un groupement acétyle ou un groupement aromatique. De préférence on utilise pour protéger la fonction carboxyle de l'acide aminé C-terminal, une substitution par un groupement R₂ de type chaîne alkyle de C₁ à C₃₀, ou un groupement NH2, NHY ou NYY avec Y représentant une chaîne alkyle de C₁ à C₄.

Le peptide selon l'invention peut être protégé au niveau de l'extrémité N-terminale, C-terminale ou au niveau des deux extrémités.

Ainsi, l'invention concerne une composition telle que définie précédemment, caractérisée par le fait que le peptide de séquence SEQ ID n° 4 à SEQ ID n° 7 est sous forme protégée ou non.

Le peptide selon l'invention peut être obtenu soit par synthèse chimique classique (en phase solide ou en phase homogène liquide), soit par synthèse enzymatique (Kullman et al., J. Biol. Chem. 1980, 225, 8234), à partir d'acides aminés constitutifs.

Le peptide selon l'invention peut être d'origine naturelle ou synthétique. Préférentiellement selon l'invention, le peptide est d'origine synthétique, obtenu par synthèse chimique.

Selon l'invention, l'agent actif peut être un peptide unique, un mélange de peptides ou de dérivés de peptides.

Le peptide selon l'invention est avantageusement solubilisé dans un ou plusieurs solvants physiologiquement adaptés, tels que l'eau, le glycérol, l'éthanol, le propanediol, le butylène glycol, le dipropylène glycol, les diglycols éthoxylés ou propoxylés, les polyols cycliques ou tout mélange de ces solvants. Le peptide dilué est ensuite stérilisé par filtration stérile.

Après cette étape de dilution, le peptide peut être encapsulé ou inclus dans un vecteur cosmétique ou pharmaceutique tel que les liposomes ou toute autre microcapsule utilisée dans le domaine de la cosmétique ou adsorbé sur des polymères organiques poudreux, des supports minéraux comme les talcs et bentonites.

On entend par «physiologiquement adaptés » que le solvant choisi est approprié pour entrer en contact avec la peau sans provoquer de réactions de toxicité ou d'intolérance.

Le peptide selon l'invention peut être utilisé à titre de médicament.

L'invention a pour second objet une composition cosmétique ou pharmaceutique, et notamment dermatologique, comprenant, dans un milieu physiologiquement adapté, un peptide selon l'invention en tant qu'agent actif activateur de la SIRT6 humaine.

Selon un mode de réalisation avantageux de l'invention, l'agent actif selon l'invention est présent dans les compositions de l'invention à une concentration comprise entre 10⁻⁹ M et 10⁻³ M environ, et préférentiellement à une concentration comprise entre 2.10⁻⁸ M et 10⁻⁵ M par rapport au poids total de la composition finale.

Cette fourchette de concentrations représente la quantité efficace d'agent actif correspondant à la quantité nécessaire pour obtenir le résultat recherché, à savoir, activer la SIRT6, diminuer les dégradations de l'ADN et améliorer la maintenance des télomères.

De manière préférée, la composition selon l'invention se présente sous une forme adaptée à l'application par voie topique comprenant un milieu physiologiquement adapté pour la peau. Par « physiologiquement adapté », on entend des milieux qui conviennent à une utilisation en contact avec la peau ou les phanères humains, sans risque de toxicité, d'incompatibilité, d'instabilité, de réponse allergique et autres effets secondaires.

On entend par « application topique », le fait d'appliquer ou d'étaler l'agent actif selon l'invention, ou une composition le contenant, à la surface de la peau.

Les compositions destinées à être appliquées sur la peau peuvent se présenter sous forme de solution aqueuse ou hydro-alcoolique, d'émulsion eau dans huile ou huile dans eau, de microémulsion, de gel aqueux ou anhydre, de sérum, ou encore de dispersion de vésicule, de patch, de crème, de spray, d'onguent, de pommade, de lotions, de colloïde, de solution, de suspension ou autres.

Ces compositions pourront notamment se présenter sous forme d'une solution aqueuse, hydro-alcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

L'ensemble de ces compositions comprennent, en outre, tout additif communément utilisé dans le domaine d'application envisagé ainsi que les adjuvants nécessaires à leur formulation, tels que des co-solvants (éthanol, glycérol, alcool benzylique, humectant...), des épaississants, des diluants, des émulsionnants, des anti-oxydants, des colorants, des filtres solaires, des pigments, des charges, des conservateurs, des parfums, des absorbeurs d'odeur, des huiles essentielles, des oligo-éléments, des acides gras essentiels, des tensioactifs, des polymères filmogènes, des filtres chimiques ou minéraux, des agents hydratants ou des eaux thermales etc. On peut par exemple citer des polymères hydrosolubles de type polymère naturel, tels que les polysaccharides, ou polypeptides, des dérivés cellulosiques de type méthylcellulose ou hydroxypropylcellulose, ou encore des polymères synthétiques, polaxamères, carbomères, siloxanes, PVA ou PVP et notamment les polymères vendus par la société ISP.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, être présents à des concentrations allant de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Il est bien entendu que l'agent actif selon l'invention peut être utilisé seul ou bien en association avec d'autres agents actifs.

Avantageusement, les compositions utilisables selon l'invention contiennent, en outre, au moins un autre agent actif destiné à favoriser l'action de l'agent actif selon l'invention, et destinés, notamment, à la prévention et/ou au traitement des désordres liés au vieillissement.

On peut citer, de manière non limitative, les classes d'ingrédients suivantes : d'autres agents actifs peptidiques, des extraits de végétaux, des agents cicatrisants, anti-âge, antirides, apaisants, anti-radicalaires, anti-UV, des agents stimulant la synthèse de macromolécules dermiques ou le métabolisme énergétique, des agents hydratants, antibactériens, antifongiques, anti-inflammatoires, anesthésiques, des agents modulant la différenciation, la pigmentation ou la dépigmentation cutanée, des agents stimulant la pousse des ongles ou des cheveux.

Préférentiellement, on utilisera un agent anti-radicalaire ou antioxydant, ou un agent stimulant la synthèse de macromolécules dermiques, ou encore un agent stimulant le métabolisme énergétique. Dans un mode plus particulier de réalisation, la composition selon l'invention comprendra, outre le peptide selon l'invention,
- au moins un composé activateur du cytochrome c, et/ou,
- au moins un composé hydratant, tel qu'un composé activateur des aquaporines et/ou ;
- au moins un composé activateur des sirtuines et en particulier les peptides cités dans les brevets FR 2 883754, US11/910,098, EP 1868631 et/ou,
- au moins un composé qui augmente l'adhésion cellulaire et/ou,
- au moins un composé qui augmente la production de protéines matricielles telles que le collagène, fibronectine, laminine, glycosaminoglycanes et/ou,
- au moins un composé modulateur de l'activité du protéasome et/ou,
- au moins un composé modulateur du rythme circadien et/ou,
- au moins un composé modulateur des protéines HSP et/ou,
- au moins un composé qui augmente l'énergie cellulaire et/ou,
- au moins un composé modulant la pigmentation de la peau et/ou,
- au moins un composé activateur du coenzyme Q10 et/ou,
- au moins un composé améliorant la fonction barrière, tels que un composés activateur de la transglutaminase ou de la HMG-CoA réductase et/ou,
- au moins un composé protecteur de la mitochondrie et/ou,
- au moins un composé protecteur ou modulateur des cellules somatiques adultes de l'épiderme ou du derme et/ou,
- au moins un composé protecteur ou réparateur des dégradations de l'ADN.

Lesdits composés ci-dessus peuvent être d'origine naturelle, ou synthétique. Lesdits composés ci-dessus peuvent être des agents actifs de nature peptidique et plus particulièrement des peptides synthétiques. Lesdits composés ci-dessus peuvent également être choisi parmi des extraits végétaux ou de microorganisme, et plus particulièrement des hydrolysats peptidiques de plantes ou de microorganismes.

Indépendamment de leurs fonctions, les autres agents actifs associés à l'agent actif selon l'invention dans la composition pourront avoir des structures chimiques très diverses. On peut citer de manière non limitative, les peptides, la vitamine C et ses dérivés, les vitamines du groupe B, la DHEA (dihydroépiandrostérone), les phytostérols, l'acide salicylique et ses dérivés, les rétinoïdes, les flavonoïdes, les amines de sucres, les azoles, les sels métalliques, les extraits peptidiques d'origine naturelle ou encore les polymères naurels ou de synthèse.

L'invention a encore pour objet une composition pharmaceutique comprenant, dans un milieu physiologiquement acceptable, le peptide selon l'invention à titre de médicament. La composition pharmaceutique selon l'invention permettra d'améliorer les symptômes dermatologiques liés à l'âge ou au photo-vieillissement prématuré, parmi lesquels on peut citer la xérose, les dépigmentations ou au contraire les tâches pigmentaires, les kératoses, etc.

Avantageusement, selon cette forme de l'invention, les compositions seront appropriées à une administration orale pour un usage pharmaceutique. Ainsi, les compositions pourront notamment se présenter sous forme de comprimés, capsules, gélules, pâtes à mâcher, poudres à consommer telles quelles ou à mélanger extemporanément avec un liquide, sirop, gel, et toute autre forme connue de l'homme du métier. Elles contiendront des excipients de formulation appropriés, tels que colorants, édulcorants, aromatisants, agents de charge, liants, conservateurs.

L'invention a pour troisième objet l'utilisation d'une composition cosmétique comprenant le peptide selon l'invention en tant qu'agent actif, pour prévenir et/ou réparer les dégradations de l'ADN.

On entend « agent actif pour prévenir et/ou réparer les dégradations de l'ADN » un peptide capable de limiter la dégradation de l'ADN ou de favoriser la réparation des dommages dus à des réactions photochimiques entre les bases de l'ADN.

L'invention a pour quatrième objet l'utilisation d'une composition cosmétique comprenant le peptide selon l'invention en tant qu'agent actif, pour améliorer la maintenance des télomères et diminuer la sénescence cellulaire.

On entend « agent actif pour améliorer la maintenance des télomères et diminuer la sénescence cellulaire» un peptide capable d'augmenter la synthèse de protéines spécifiquement associées aux télomères et participant à leur stabilité, telles que TRF2 et SIRT6.

L'invention a pour cinquième objet l'utilisation d'une composition cosmétique comprenant le peptide selon l'invention en tant qu'agent actif, pour augmenter l'expression des marqueurs de différenciation des kératinocytes et pour favoriser l'expression des protéines de la matrice extracellulaire par les fibroblastes de la peau.

Ces propriétés particulières de l'agent actif selon l'invention, permettent d'améliorer la qualité du derme et donc la fermeté de la peau, et d'optimiser la fonction barrière de l'épiderme.

L'invention a pour sixième objet l'utilisation d'une composition cosmétique comprenant le peptide selon l'invention en tant qu'agent actif, pour protéger la peau contre tous types d'agressions extérieures.

On entend par l'expression « agressions extérieures », les agressions que peut produire l'environnement. A titre d'exemple, on peut citer des agressions telles que la pollution, les UV, ou encore les produits à caractère irritant tels que les tensioactifs, les conservateurs ou les parfums, les agressions mécaniques, telles que les abrasions, le rasage ou l'épilation. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldéhyde), ou bien encore la fumée de cigarette.

En particulier, l'invention a pour objet l'utilisation d'une composition cosmétique comprenant une quantité efficace de peptide selon l'invention, pour prévenir ou traiter les dommages causés à la peau par des expositions aux rayonnements UV et par des stress oxydatifs.

L'invention a pour septième objet un procédé de traitement cosmétique caractérisé en ce que l'on applique topiquement sur la peau à traiter une composition comprenant une quantité efficace d'agent actif selon l'invention pour prévenir et/ou traiter les signes cutanés du vieillissement et du photo-vieillissement.

Par manifestations cutanées du vieillissement, on entend toutes modifications de l'aspect extérieur de la peau et des phanères dues au vieillissement comme, par exemple, les rugosités superficielles de la couche cornée, les rides et ridules, mais également toute modification interne de la peau qui ne se traduit pas systématiquement par un aspect extérieur modifié comme, par exemple, l'amincissement du derme ou toute autre dégradation interne de la peau consécutive à une exposition aux rayonnements UV.

En particulier, l'invention se rapporte à un procédé de traitement cosmétique destiné à protéger la peau contre les agressions dues aux rayonnements UV.

D'autres avantages et caractéristiques de l'invention apparaîtront mieux à la lecture des exemples donnés à titre illustratif et non limitatif.

### Liste des figures

Figure 1 : Alignement des séquences peptidiques des protéines SIRT humaines (l'alignement a été réalisé à l'aide du programme d'alignement de séquences peptidiques multiples ClustalW2 de l'European Bioinformatics Institute)
Figure 2 : Quantification de l'immunomarquage de la sirtuine 6 (SIRT6) dans des fibroblastes humains normaux, traités 24 heures par le peptide SEQ ID n°5.

### Exemple 1 : Mise en évidence de l'effet activateur des peptides SEQ ID n°5 et SEQ D n°7 sur l'expression de la sirtuine 6

Le but de cette étude est de déterminer l'influence des peptides SEQ ID n°5 et SEQ ID n°7 sur l'expression de la Sirtuine 6 dans la peau humaine. Pour cela des marquages spécifiques par immunofluorescence ont été réalisés sur culture de kératinocytes humains normaux (KHN) et sur des cultures de fibroblastes humains normaux.

Protocole : Des KHN ou des fibroblastes humains normaux en culture sont traités une fois par jour avec une solution à 10⁻⁶ M ou à 3.10⁻⁶ M de peptide SEQ ID n°5 ou de peptide SEQ ID n°7.

Des études de traitement à court terme, pendant 24, 48 et 72 heures ont été réalisées.

Des études de traitement à long terme ont également été réalisées, entre le passage 5 et le passage 17 (soit 12 passages) pour les fibroblastes, et entre le passage 3 et le passage 5 (soit 2 subcultures) pour les KHN.

Pour l'immunomarquage par l'anticorps anti SIRT6, les cellules sont lavées et fixées au paraformaldéhyde à 3,7% pendant 10 minutes.

Les cellules sont ensuite incubées en présence d'un anticorps spécifique anti SIRT6 (Abcam, ref. ab62738, polyclonal de lapin), puis d'un anticorps secondaire adapté, couplé à un marqueur fluorescent. Après montage dans un milieu *ad hoc,* les lames sont observées au microscope à épifluorescence (Nikon Eclipse E 80i microscope).

L'intensité de la fluorescence est quantifiée par analyse d'image à l'aide du logiciel Image-Pro Analyser version 5.

Résultats : Dans toutes les conditions testées, on observe une fluorescence plus intense dans les cultures traitées par le peptide SEQ ID n°5 et par le peptide SEQ ID n°7 à 10⁻⁶ M ou à 3.10⁻⁶ M que dans les conditions contrôle.

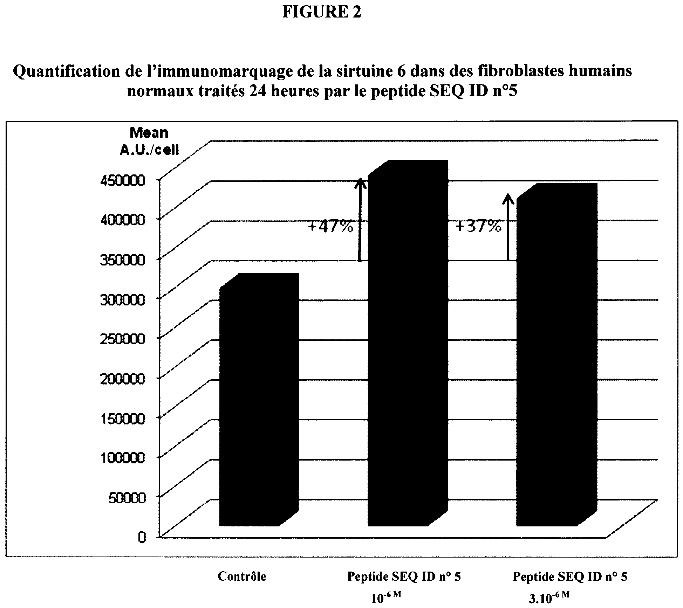

Dans les fibroblastes, on observe une augmentation maximum de 47% de la fluorescence dans les cellules traitées pendant 24 heures par 10⁻⁶ M de peptide SEQ ID n°5, par rapport aux cellules contrôle (Figure 2).

Dans les KHN, on observe une augmentation maximum de 35% de la fluorescence dans les cellules traitées pendant 72 heures par 3.10⁻⁶ M de peptide SEQ ID n°5, par rapport aux cellules contrôle. L'augmentation de la fluorescence est dose-dépendante pendant les 72 premières heures.

D'autre part, l'augmentation de l'expression de SIRT6 se maintient lors d'un traitement à long terme, pour les deux types de cellules testées.

Conclusions : Les peptides SEQ ID n°5 et SEQ ID n°7 augmentent très significativement l'expression de la sirtuine 6 dans les fibroblastes humains normaux et les KHN, dans des cultures à court terme. De plus, l'effet de stimulation de l'expression de la sirtuine 6 se maintien à long terme.

### Exemple 2 : Mise en évidence de l'effet activateur du peptide SEQ ID n°4 sur l'expression de la protéine TRF2

Le but de cette étude est de déterminer l'influence du peptide SEQ ID n°4 sur l'expression de la protéine TRF2 dans la peau humaine, une protéine spécifiquement associée aux télomères et impliquée dans leur maintenance. Pour cela des marquages spécifiques par immunofluorescence ont été réalisés sur culture de kératinocytes humains normaux (KHN) et sur des cultures de fibroblastes humains normaux à long terme.

Protocole : Des KHN ou des fibroblastes humains normaux en culture sont traités une fois par jour avec une solution à 10⁻⁶ M ou à 3.10⁻⁶ M de peptide SEQ ID n°4, entre le passage 5 et le passage 17 (soit 12 passages) pour les fibroblastes et entre le passage 1 et le passage 2 (soit 1 passage et 10 jours de traitement) pour les KHN.

Pour l'immunomarquage par l'anticorps anti TRF2, les cellules sont lavées et fixées au paraformaldéhyde à 3,7% pendant 10 minutes.

Les cellules sont ensuite incubées en présence d'un anticorps spécifique anti TRF2 (Abcam, ref. ab13579 monoclonal de souris), puis d'un anticorps secondaire adapté, couplé à un marqueur fluorescent. Après montage dans un milieu *ad hoc,* les lames sont observées au microscope à épifluorescence (Nikon Eclipse E 80i microscope).

L'intensité de la fluorescence est quantifiée par analyse d'image à l'aide du logiciel Image-Pro Analyser version 5.

Résultats : Dans toutes les conditions testées, on observe une fluorescence plus intense dans les cultures traitées par le peptide SEQ ID n°4 à 10⁻⁶ M ou à 3.10⁻⁶ M que dans les conditions contrôle.

Dans les fibroblastes, on observe une augmentation maximum de 63% de la fluorescence dans les cellules traitées pendant 12 subcultures par 10⁻⁶ M de peptide SEQ ID n°4, par rapport aux cellules contrôle. L'augmentation de la fluorescence est dose-dépendante.

Dans les KHN, on observe une augmentation maximum de 39% de la fluorescence dans les cellules traitées pendant 10 jours par 3.10⁻⁶ M de peptide SEQ ID n°4, par rapport aux cellules contrôle. L'augmentation de la fluorescence est dose-dépendante.

Conclusions : Le peptide SEQ ID n°4 augmente très significativement l'expression de la protéine TRF2 dans les fibroblastes humains normaux et les KHN, de façon dose dépendantes, dans des cultures à long terme.

### Exemple 3: Mise en évidence de l'effet activateur du peptide SEQ ID n°5 sur la différenciation épidermique et la fonction barrière de l'épiderme

Le but de cette étude est de déterminer l'influence du peptide SEQ ID n°5 sur la différenciation épidermique. Pour cela, l'expression des principaux marqueurs de la différenciation épidermique, exprimés spécifiquement dans les kératinocytes des couches suprabasales cultivés à long terme, a été étudiée. Les marqueurs testés sont la transglutaminase 1 et l'involucrine.

Protocole : Des KHN en culture sont traités une fois par jour avec une solution à 10⁻⁶ M ou à 3.10⁻⁶ M de peptide SEQ ID n°5, entre le passage 1 et le passage 3 (soit 2 passages et 11 jours de traitement).

Les cellules sont ensuite lavées et fixées. Après démasquage des sites spécifiques, les cellules sont incubées en présence d'un anticorps spécifique dirigé contre la TG1 (TEBU, ref. sc-25786, polyclonal de lapin), d'un anticorps spécifique dirigé contre l'involucrine (Novocastra NCL-INV, mouse monoclonal, clone SY5), puis incubées en présence d'un anticorps secondaire adapté, couplé à un marqueur fluorescent. Pour une plus grande facilité d'observation, les noyaux des cellules peuvent être contre-colorés par le DAPI (4',6' Di Amidino-2-Phényl Indole), une molécule fluorescente bleue capable de se lier fortement à l'ADN). Après montage dans un milieu *ad hoc,* les lames sont observées au microscope à épifluorescence (Nikon Eclipse E 80i microscope).

Résultats : On observe une fluorescence plus intense dans les cultures et sur les coupes de peaux traitées par le peptide SEQ ID n°5 à 10⁻⁶ M ou à 3.10⁻⁶ M que dans les conditions contrôle.

Conclusions : Le peptide SEQ ID n°5 à 10⁻⁶ M ou à 3.10⁻⁶ M améliore la différenciation des KHN et ce faisant optimise la fonction barrière de l'épiderme.

### Exemple 4 : Mise en évidence de l'effet activateur du peptide SEQ ID n°5 sur l'expression des molécules de la matrice extracellulaire dermique

Le but de cette étude est de déterminer l'influence du peptide SEQ ID n°5 sur l'expression des molécules de la matrice extracellulaire dermique. Pour cela, l'expression des collagènes I et III dans des fibroblastes humains normaux cultivés à long terme a été étudiée.

Protocole : Des fibroblastes humains normaux sont traités une fois par jour avec une solution à 10⁻⁶ M ou à 3.10⁻⁶ M de peptide SEQ ID n°5, entre le passage 5 et le passage 17 (soit 12 passages).

Les cellules sont ensuite lavées et fixées au méthanol à froid pendant 5 minutes. Après démasquage des sites spécifiques, les cellules sont incubées en présence d'un anticorps spécifique dirigé contre le collagène I (TEBU, réf. 600-401-103, polyclonal de lapin) ou contre le collagène III (TEBU, réf. 600-401-105, polyclonal de lapin), puis incubées en présence d'un anticorps secondaire adapté, couplé à un marqueur fluorescent. Après montage dans un milieu *ad hoc,* les lames sont observées au microscope à épifluorescence (Nikon Eclipse E 80i microscope).

Résultats : On observe une fluorescence plus intense dans les cultures et sur les coupes de peaux traitées par le peptide SEQ ID n°5 à 10⁻⁶ M ou à 3.10⁻⁶ M que dans les conditions contrôle.

Conclusions : Le peptide SEQ ID n°5 à 10⁻⁶ M ou à 3.10⁻⁶ M appliqué à long terme, augmente l'expression du collagène I et du collagène III, deux protéines essentielles de la matrice extracellulaire dermique.

### Exemple 5 : Mise en évidence de l'effet du peptide SEQ ID n°5 sur les dommages causés à l'ADN par une irradiation UV

Le but de cette étude est de déterminer l'effet protecteur du peptide SEQ ID n°5 sur les dommages causés à l'ADN par une irradiation UV. Pour cela, un test comètes, qui permet de quantifier les dommages causés à l'ADN au niveau cellulaire, a été réalisé.

Protocole : Des fibroblastes humains normaux sont mis en culture pendant 24 heures avec le peptide de séquence SEQ ID n° 5 à une concentration de 10⁻⁶ M ou 3.10⁻⁶ M, puis irradiées avec des rayonnements UVB à raison de 60 mJ/ cm², puis traitées de nouveau pendant 24 h par le peptide à une concentration de 10⁻⁶ M ou 3.10⁻⁶ M.

Une condition contrôle est réalisée en l'absence de traitement.

Les cellules sont ensuite détachées de leur support par de la trypsine, puis centrifugées à 1200 tour/min pendant 10 minutes afin de les concentrer et les dénombrer.

Un nombre défini de cellules (25 000 cellules) est ensuite inclut dans un gel d'agarose Low Melting à 0,75 %, puis déposé sur une lame de verre préalablement recouverte d'agarose à 1 %. Les lames sont ensuite immergées dans une solution de lyse pendant 1h30 à 4 °C, puis dans une solution alcaline pendant 20 min à 4 °C. Les cellules sont ainsi lysées et l'ADN dénaturé. Les lames sont plongées dans une solution d'électrophorèse avant d'appliquer un champ électrique (20 V - 250 mA). L'ADN ainsi dénaturé est soumis à une migration au sein du gel d'agarose à 4 °C, pendant 30 min. L'application d'un colorant fluorescent de l'ADN sur les lames, l'iodure de propidium à 2 µg/ ml, pendant 20 min, permet d'observer au microscope l'ADN apparaissant sous forme de queue de comètes dans le cas où il a été endommagé.

Un logiciel de quantification permet de déterminer le « Tail Moment » moyen (ou longueur de la queue de la comète) appliqué à chaque condition testée. Ce paramètre renseigne sur le niveau d'endommagement de l'ADN : plus il est élevé, plus les dégradations de l'ADN sont importantes.

Résultats : Les résultats montrent une diminution de 24,8 % du Tail Moment lorsque les cellules sont traitées par le peptide de SEQ ID n°5 à 3.10⁻⁶ M, comparés aux conditions contrôle.

Conclusion : L'ADN des cellules traitées puis soumises à une irradiation UVB a subi moins de dommages que 14ADN des cellules contrôle. Ces résultats confirment l'effet protecteur préventif et curatif vis à vis des rayonnements UVB du peptide de séquence SEQ ID n°5.

### Exemple 6 : Mise en évidence de l'effet protecteur du peptide SEQ ID n°5 lors d'un stress oxydatif

Le but de cette étude est de déterminer l'effet protecteur du peptide SEQ ID n°5 sur les kératinocytes lors d'un stress oxydatifs. Pour cela, l'expression de la Sirtuine 6 a été évaluée qualitativement et quantitativement par des immunomarquages spécifiques après un stress oxydatif par l'H₂O₂.

Protocole : Des KHN en culture sont traités pendant 24 heures, avec une solution à 10⁻⁶ M ou 3.10⁻⁶ M de peptide SEQ ID n° 5. Les cellules sont ensuite incubées en présence de H₂O₂ à 2 mM, rincées puis traitées pendant encore 24 heures, avec une solution à 10⁻⁶ M ou 3.10⁻⁶ M de peptide SEQ ID n° 5. Un contrôle non traité et non soumis au stress H₂O₂ (contrôle 0), ainsi qu'un contrôle non traité mais soumis au stress H₂O₂ (contrôle 1) sont réalisés.

Pour l'immunomarquage par l'anticorps anti SIRT6, les cellules sont lavées et fixées au paraformaldéhyde à 3,7% pendant 10 minutes.

Les cellules sont ensuite incubées en présence d'un anticorps spécifique anti SIRT6 (Abcam, réf. ab62738, polyclonal de souris), puis d'un anticorps secondaire adapté, couplé à un marqueur fluorescent. Après montage dans un milieu *ad hoc,* les lames sont observées au microscope à épifluorescence (Nikon Eclipse E 80i microscope).

L'intensité de la fluorescence est quantifiée par analyse d'image à l'aide du logiciel Image-Pro Analyser version 5.

Résultats :_L'analyse quantitative montre une augmentation de respectivement 16% et 20% de l'expression de SIRT6, lorsque les KHN sont traités par le peptide SEQ ID n°5 à 10⁻⁶ M ou 3.10⁻⁶ M, et soumis à un stress H₂O₂, par rapport au contrôle 1.

Conclusions : Les cellules traitées par le peptide SEQ ID n°5, préventivement et postérieurement à un stress oxydatif, ont un contenu en protéine SIRT6 supérieur à celui des cellules contrôle. Ces résultats confirment que le peptide de séquence SEQ ID n°5 favorise la protection des KHN lors d'un stress oxydatif.

### Exemple 7 : Préparation de compositions

### 1 - Crème protection solaire:

| Noms commerciaux | Noms INCI | % massique |
|---|---|---|
| PHASE A | | |
| Eau déminéralisée | Aqua (Water) | qsp |
| Pemulen TR1 | Acrylates/C10-30 Alkyl Acrylate Crosspolymer | 0,40 |
| Glycerine | Glycerin | 3,00 |
| Nipastat Sodium | Sodium Methylparaben (and) Sodium Ethylparaben (and) Sodium Butyl paraben (and) Sodium Propylparaben (and) Sodium Isobutylparaben | 0,15 |

| PHASE B | | |
|---|---|---|
| Parsol MCX | Ethylhexyl Methoxycinnamate | 7,50 |
| Eusolex 4360 | Benzophenone-3 | 3,00 |
| Parsol 1789 | Butyl Methoxydibenzoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| PHASE C | | |
|---|---|---|
| TEA | Triethanolamine | 0,20 |

| PHASE D | | |
|---|---|---|
| Peptide SEQ ID n° 4 | | 3.10⁻⁶ M |

| Noms commerciaux | Noms INCI | % massique |
|---|---|---|
| Parfum | Parfum (Fragrance) | qsp |
| Colorant | | qsp |

Les constituants de la phase A et de la phase B sont chauffés séparément entre 70°C et 75°C. La phase B est émulsionnée dans la phase A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

### 2-Crème anti-âge :

| ***Noms commerciaux*** | ***Noms INCI*** | ***% massique*** |
|---|---|---|
| ***Phase A*** | | |
| Montanov 68 | Cetearyl Alcohol (and) Cetearyl Glucoside | 6,00 |
| Squalane | Squalane | 3,00 |
| Cetiol SB 45 | Butyrospermum Parkii ( Shea Butter) | 2,00 |
| Waglinol 250 | Cetearyl Ethylhexanoate | 3,00 |
| Amerchol L- 101 | Mineral Oil (and) Lanolin Alcohol | 2,00 |
| Abil 350 | Dimethicone | 1,50 |
| BHT | BHT | 0,01 |
| Coenzyme Q10 | Ubiquinone | 0,10 |

| ***Phase B*** | | |
|---|---|---|
| Huile d'Avocat | Persea Gratissima (Avocado) Oil | 1,25 |
| Phenonip | Phenoxyethanol (and) Methylparaben (and) Ethylparaben (and) Butylparaben (and) Propylparaben (and) Isobutylparaben | 0,75 |

| ***Phase C*** | | |
|---|---|---|
| Eau déminéralisée | Aqua (Water) | qsp |
| Butylene Glycol | Butylene Glycol | 2,00 |
| Glucam E10 | Methyl Gluceth-10 | 1,00 |

| ***Noms commerciaux*** | ***Noms INCI*** | ***% massique*** |
|---|---|---|
| Allantoin | Allantoin | 0,15 |
| Carbopol Ultrez 10 | Carbomer | 0,20 |

| ***Phase D*** | | |
|---|---|---|
| TEA | Triethanolamine | 0,18 |

| ***Phase E*** | | |
|---|---|---|
| Peptide SEQ ID n°5 | | 1.10⁻⁶ M |
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| Parfum | Parfum (Fragrance) | qsp |
| Colorant | | qsp |

Préparer et fondre la phase A à 65-70°C. Chauffer la phase C à 65-70°C. La phase B est ajoutée à la phase A juste avant d'émulsionner A dans B. A environ 45°C, le carbomer est neutralisé par addition de la phase D. La phase E est ensuite additionnée sous légère agitation et le refroidissement est poursuivi jusqu'à 25°C. La phase F est alors additionnée si souhaité.

### 3 -Crème protectrice de jour :

| ***Noms commerciaux*** | ***Noms INCI*** | ***% massique*** |
|---|---|---|
| ***Phase A*** | | |
| Emulium Delta | Cetyl alcohol (and) Glyceryl Stearate (and) PEG-75 Stearate (and) Ceteth-20 (and) Steareth-20 | 4,00 |
| Lanette O | Cetearyl Alcohol | 1,50 |
| D C 200 Fluid/100cs | Dimethicone | 1,00 |
| DUB 810C | Coco Caprylate/Caprate | 1,00 |
| DPPG | Propylene Glycol Dipelargonate | 3,00 |
| DUB DPHCC | Dipentaerythrityl Hexacaprylate/Hexacaprate | 1,50 |
| Cegesoft PS6 | Vegetable Oil | 1,00 |
| Vitamine E | Tocopherol | 0,30 |
| Phenonip | Phenoxyethanol (and) Methylparaben (and) Ethylparaben (and) Butylparaben (and) Propylparaben (and) Isobutylparaben | 0,70 |

| ***Phase B*** | | |
|---|---|---|
| Eau déminéralisée | Aqua | qsp 100 |
| Glycerine | Glycerin | 2,00 |
| Carbopol EDT 2020 | Acrylates/C10-30Alkyl Acrylate Crosspolymer | 0,15 |
| Keltrol BT | Xanthan Gum | 0,30 |

| ***Phase C*** | | |
|---|---|---|
| Sodium Hydroxide (sol.à 10%) | Sodium Hydroxide | 0,30 |

| ***Phase D*** | | |
|---|---|---|
| Eau déminéralisée | Aqua | 5,00 |
| Stay-C 50 | Sodium Ascorbyl Phosphate | 0,50 |

| ***Phase E*** | | |
|---|---|---|
| Butylene Glycol | Butylene Glycol | 2,00 |
| Dekaben CP | Chlorphenesin | 0,20 |

| ***Phase F*** | | |
|---|---|---|
| GP4G | Water (and) Artemia Extract | 1,00 |
| Peptide SEQ ID n°5 | | 2.10⁻⁶ M |

Préparer la phase A et chauffer à 75°C sous agitation. Préparer la phase B en dispersant le carbopol, puis la gomme xanthane sous agitation. Laisser reposer. Chauffer à 75°C.

A température, émulsionner A dans B sous agitation rotor-stator. Neutraliser avec la phase C sous agitation rapide. Après refroidissement à 40°C, additionner la phase D, puis la phase E. Le refroidissement est poursuivi sous agitation légère et la phase F rajoutée.

## Revendications

1. Peptide dérivé de la séquence peptidique de régions hautement conservées des protéines SIRT humaines, **caractérisé en ce qu'**il correspond à une des séquences suivantes :
(SEQ ID n°4) Gly-Ala-Gly-Val-Ser-Ala-Glu
(SEQ ID n°5) Gly-Ala-Gly-Val-Ser-Ala-Glu-NH₂
(SEQ ID n°6) Thr-Gln-Asn-Ile-Asp-Glu-Leu
(SEQ ID n°7) Thr-Gln-Asn-Ile-Asp-Glu-Leu-NH₂

2. Peptide selon la revendication 1, **caractérisé en ce qu'**il est solubilisé dans un ou plusieurs solvants physiologiquement adaptés, comme l'eau, le glycérol, l'éthanol, le propanediol, le propylène glycol, le butylène glycol, le dipropylène glycol, les diglycols éthoxylés ou propoxylés, les polyols cycliques, la vaseline, une huile végétale ou tout mélange de ces solvants.

3. Peptide selon l'une des revendications 1 ou 2, utilisé à titre de médicament.

4. Composition cosmétique, **caractérisée en ce qu'**elle comprend, dans un milieu physiologiquement adapté, au moins un peptide tel que défini dans l'une des revendications 1 à 2, en tant qu'agent actif activateur de la SIRT6, utilisé seul ou en association avec au moins un autre agent actif.

5. Composition selon la revendication 4, **caractérisée en ce que** ledit peptide est présent à une concentration comprise entre 10⁻⁹ M et 10⁻³ M par rapport au poids total de la composition finale.

6. Composition selon l'une des revendications 4 ou 5, **caractérisée en ce qu'**elle est destinée à une administration par voie topique.

7. Composition selon l'une quelconque des revendications 4 à 6, **caractérisée en ce qu'**elle contient en outre, au moins un autre agent actif choisi parmi la vitamine C, les vitamines du groupe B, la DHEA (dihydroépiandrostérone), les phytostérols, l'acide salicylique, les rétinoïdes, les flavonoïdes, les amines de sucres, les azoles, les sels métalliques ou les hydrolysats peptidiques de plantes ou de microorganismes.

8. Utilisation cosmétique d'une composition comprenant le peptide tel que défini dans l'une des revendications 1 à 2 et un milieu physiologiquement adapté, pour prévenir et/ou réparer les dégradations de l'ADN.

9. Utilisation cosmétique d'une composition comprenant le peptide tel que défini dans l'une des revendications 1 à 2 et un milieu physiologiquement adapté, pour améliorer la maintenance des télomères.

10. Utilisation cosmétique d'une composition comprenant le peptide tel que défini dans l'une des revendications 1 à 2 et un milieu physiologiquement adapté, pour augmenter l'expression des marqueurs de différenciation des kératinocytes et pour favoriser l'expression des protéines de la matrice extracellulaire par les fibroblastes de la peau.

11. Utilisation cosmétique d'une composition comprenant le peptide tel que défini dans l'une des revendications 1 à 2 et un milieu physiologiquement adapté pour protéger la peau contre tout type d'agressions extérieures.

12. Utilisation selon la revendication 11, **caractérisée en ce que** les agressions extérieures sont provoquées par les rayonnements UV.

13. Utilisation selon la revendication 11, **caractérisée en ce que** les agressions extérieures sont provoquées par les stress oxydatifs.

14. Procédé de traitement cosmétique destiné à prévenir et/ou traiter les signes cutanés du vieillissement et du photo-vieillissement, **caractérisé en ce que** l'on applique topiquement sur la peau à traiter une composition selon l'une des revendications 4 à 7.

## Patentansprüche

1. Peptid, das von der Peptidsequenz von stark konservierten Regionen von humanen SIRT-Proteinen abgeleitet ist,
**dadurch gekennzeichnet, dass** es einer der folgenden Formeln entspricht:
(SEQ ID Nr. 4) Gly-Ala-Gly-Val-Ser-Ala-Glu
(SEQ ID Nr. 5) Gly-Ala-Gly-Val-Ser-Ala-Glu-NH₂
(SEQ ID Nr. 6) Thr-Gln-Asn-Ile-Asp-Glu-Leu
(SEQ ID Nr. 7) Thr-Gln-Asn-Ile-Asp-Glu-Leu-NH₂.

2. Peptid nach Anspruch 1, **dadurch gekennzeichnet, dass** es in einem oder mehreren geeigneten physiologisch akzeptablen Lösungsmitteln, wie Wasser, Glycerin, Ethanol, Propandiol, Propylenglykol, Butylenglykol, Dipropylenglykol, ethoxylierte oder propoxylierte Diglykole, cyclische Polyole, Vaseline, ein pflanzliches Öl oder ein beliebiges Gemisch dieser Lösungsmittel, solubilisiert ist.

3. Peptid nach einem der Ansprüche 1 oder 2, das als Arzneimittel verwendet wird.

4. Kosmetische Zusammensetzung, **dadurch gekennzeichnet, dass** sie mindestens ein wie in einem der Ansprüche 1 bis 2 definiertes Peptid als Aktivatorwirkstoff für SIRT6, allein oder in Verbindung mit mindestens einem anderen Wirkstoff verwendet, in einem physiologisch angepassten Medium umfasst.

5. Kosmetische Zusammensetzung nach Anspruch 4, **dadurch gekennzeichnet, dass** das Peptid in einer Konzentration vorliegt, die zwischen 10⁻⁹ M und 10⁻³ M liegt, bezogen auf das Gesamtgewicht der Endzusammensetzung.

6. Zusammensetzung nach einem der Ansprüche 4 oder 5, **dadurch gekennzeichnet, dass** sie für eine topische Verabreichung vorgesehen ist.

7. Zusammensetzung nach einem der Ansprüche 4 bis 6, **dadurch gekennzeichnet, dass** sie außerdem mindestens einen anderen Wirkstoff enthält, der aus Vitamin C, Vitaminen der Gruppe B, DHEA (Dihydroepiandrosteron), Phytosterinen, Salicylsäure, Retinoiden, Flavonoiden, Aminen von Zuckern, Azolen, Metallsalzen oder Peptidhydrolysaten von Pflanzen oder Mikroorganismen ausgewählt ist.

8. Kosmetische Verwendung einer Zusammensetzung, die das wie in einem der Ansprüche 1 bis 2 definierte Peptid und ein physiologisch angepasstes Medium umfasst, zum Verhindern und/oder Reparieren von DNA-Degradationen.

9. Kosmetische Verwendung einer Zusammensetzung, die das wie in einem der Ansprüche 1 bis 2 definierte Peptid und ein physiologisch angepasstes Medium umfasst, zum Verbessern der Aufrechterhaltung von Telomeren.

10. Kosmetische Verwendung einer Zusammensetzung, die das wie in einem der Ansprüche 1 bis 2 definierte Peptid und ein physiologisch angepasstes Medium umfasst, zum Erhöhen der Expression von Markern der Differenzierung von Keratinozyten und zum Begünstigen der Expression von Proteinen der extrazellulären Matrix durch Fibroblasten der Haut.

11. Kosmetische Verwendung einer Zusammensetzung, die das wie in einem der Ansprüche 1 bis 2 definierte Peptid und ein physiologisch angepasstes Medium umfasst, zum Schützen der Haut vor jeglicher Art von äußeren Angriffen.

12. Verwendung nach Anspruch 11, **dadurch gekennzeichnet, dass** die äußeren Angriffe von UV-Strahlen verursacht werden.

13. Verwendung nach Anspruch 11, **dadurch gekennzeichnet, dass** die äußeren Angriffe von oxidativen Stressen verursacht werden.

14. Verfahren zur kosmetischen Behandlung, das dazu vorgesehen ist, Zeichen der Alterung und der Lichtalterung der Haut zu verhindern und/oder zu behandeln, **dadurch gekennzeichnet, dass** eine Zusammensetzung nach einem der Ansprüche 4 bis 7 topisch auf die zu behandelnde Haut aufgebracht wird.

## Claims

1. Peptide derived from the peptide sequence of highly preserved regions of human SIRT proteins,
**characterised in that** it corresponds to one of the following sequences:
(SEQ ID NO:4) Gly-Ala-Gly-Val-Ser-Ala-Glu
(SEQ ID NO:5) Gly-Ala-Gly-Val-Ser-Ala-Glu-NH₂
(SEQ ID NO:6) Thr-Gln-Asn-Ile-Asp-Glu-Leu
(SEQ ID NO:7) Thr-Gln-Asn-Ile-Asp-Glu-Leu-NH₂

2. Peptide according to claim 1, **characterised in that** it is solubilised in one or more physiologically suitable solvents, such as water, glycerol, ethanol, propanediol, propylene glycol, butylene glycol, dipropylene glycol, ethoxylated or propoxylated diglycols, cyclic polyols, Vaseline, a plant oil or any mixture of these solvents.

3. Peptide according to one of claims 1 or 2, used by way of medication.

4. Cosmetic composition, **characterised in that** it comprises, in a physiologically suitable medium, at least one peptide as defined in one of claims 1 to 2, as an active agent activating SIRT6, used alone or in association with at least one other active agent.

5. Composition according to claim 4, **characterised in that** said peptide is present at a concentration of between 10⁻⁹ M and 10⁻³ M with respect to the total weight of the final composition.

6. Composition according to one of claims 4 or 5, **characterised in that** it is intended for topical administration.

7. Composition according to one of claims 4 to 6, **characterised in that** it also contains at least one other active agent chosen from vitamin C, vitamins in the B group, DHEA (dihydroepiandrosterone), phytosterols, salicylic acid, retinoids, flavonoids, sugar amines, azoles, metal salts or peptide hydrolysates of plants or of microorganisms.

8. Cosmetic use of a composition comprising the peptide as defined in one of claims 1 to 2 and a physiologically suitable medium, for preventing and/or repairing DNA damage.

9. Cosmetic use of a composition comprising the peptide as defined in one of claims 1 to 2 and a physiologically suitable medium, for improving the maintenance of telomeres.

10. Cosmetic use of a composition comprising the peptide as defined in one of claims 1 to 2 and a physiologically suitable medium, for augmenting the expression of keratinocyte differentiation markers and for promoting the expression of the proteins of the extracellular matrix by dermal fibroblasts.

11. Cosmetic use of a composition comprising the peptide as defined in one of claims 1 to 2 and a physiologically suitable medium, for protecting the skin against any type of external attack.

12. Use according to claim 11, **characterised in that** the external attacks are caused by UV radiation.

13. Use according to claim 11, **characterised in that** the external attacks are caused by oxidative stresses.

14. Cosmetic treatment method intended for preventing and/or treating skin signs of aging and photoaging, **characterised in that** a composition according to one of claims 4 to 7 is applied topically to the skin to be treated.


### SEQUENCE LISTING

<110> ISP Investments INC.
<120> NOUVEAUX PEPTIDES ACTIVATEURS DE LA SIRTUINE 6 ET COMPOSITION COSMETIQUE OU PHARMACEUTIQUE LES COMPRENANT
<130> Bv PCT 10-142
<150> FR 10 02698
   <151> 2010-06-29
<160> 8
<170> PatentIn version 3.3
<210> 1
   <211> 8
   <212> PRT
   <213> synthetic peptide
<220>
   <221> MOD_RES
   <222> (8)..(8)
   <223> AMIDATION
<400> 1
   Glu Ile His Gly Ser Leu Phe Lys
   1               5
<210> 2
   <211> 6
   <212> PRT
   <213> Synthetic peptide
<220>
   <221> MOD_RES
   <222> (6)..(6)
   <223> AMIDATION
<400> 2
   His Gly Ser Leu Phe Lys
   1               5
<210> 3
   <211> 8
   <212> PRT
   <213> Synthetic peptide
<220>
   <221> MOD_RES
   <222> (8)..(8)
   <223> AMIDATION
<400> 3
   Leu Val Gly Ala Gly Val Ser Ala
   1               5
<210> 4
   <211> 7
   <212> PRT
   <213> Synthetic peptide
<400> 4
   Gly Ala Gly Val Ser Ala Glu
   1               5
<210> 5
   <211> 7
   <212> PRT
   <213> synthetic peptide
<220>
   <221> MOD_RES
   <222> (7)..(7)
   <223> AMIDATION
<400> 5
   Gly Ala Gly Val Ser Ala Glu
   1 5
<210> 6
   <211> 7
   <212> PRT
   <213> Synthetic peptide
<400> 6
   Thr Gln Asn Ile Asp Glu Leu
   1 5
<210> 7
   <211> 7
   <212> PRT
   <213> Synthetic peptide
<220>
   <221> MOD_RES
   <222> (7)..(7)
   <223> AMIDATION
<400> 7
   Thr Gln Asn Ile Asp Glu Leu
   1 5
<210> 8
   <211> 8
   <212> PRT
   <213> Synthetic peptide
<220>
   <221> MOD_RES
   <222> (8)..(8)
   <223> AMIDATION
<400> 8
   Val Ile Thr Gln Asn Ile Asp Ala
   1 5